# EUROPEAN PATENT SPECIFICATION

(11) **EP 1 223 992 B1**
(45) Date of publication and mention of the grant of the patent: **30.08.2006**
(21) Application number: 00989702.6
(22) Date of filing: 25.10.2000
(51) Int. Cl.: A61L 29/12, B29C 47/00, A61L 29/14, A61M 25/10

(54) **DIMENSIONALLY STABLE BALLOONS**
FORMSTABILE BALLONS
BALLONNETS STABLES SUR LE PLAN DIMENSIONNEL

(30) Priority: 25.10.1999 US 426384
(43) Date of publication of application: 24.07.2002
(73) Proprietor: Boston Scientific Limited, St. Michael, Barbados, West Indies (BB)
(72) Inventor: CHEN, John, Jianhua, Plymouth, MN 55446 (US); WANG, Lixiao, Long Lake, MN 55356 (US); WANG, Yiqun, Shijuku-ku, Tokyo 160-0023 (JP); CHIN, Albert, C., C., Newton, MA 02461 (US)
(74) Representative: Graalfs, Edo
(86) International application number: PCT/US2000/041566
(87) International publication number: WO 2001/034062

(56) References cited:
- EP-A- 0 934 755
- WO-A-00/50105
- US-A- 5 248 305

## Description

### BACKGROUND OF THE INVENTION

Medical catheters having a balloon mounted thereon are useful in a variety of medical procedures. A balloon may be used to widen a vessel into which the catheter is inserted by dilating the blocked vessel, such as in an angioplasty procedure. More significant to the present invention however, is the use of a catheter to deliver a medical device, such as a stent, into a body lumen. Some examples of stent delivery balloons are disclosed in U.S. Patent No. 5702418, and U.S. Patent No. 5797877.

In these and other medical device delivery applications, radial expansion of a balloon may be used to expand or inflate a stent at a desired positioned within the body. Using a balloon equipped catheter to deliver a stent requires precise positioning of the balloon and stent as well as a balloon with accurate and predictable expansion properties. A known drawback of many previous delivery catheters and balloons is that when a balloon is radially inflated to a desired extent, the balloon will also expand longitudinally. As a result of longitudinal expansion of a balloon during the delivery of a medical device, the balloon itself, the medical device mounted thereupon or both apparatuses may be shifted from their pre-inflation position resulting in improper delivery of the medical device.

In balloons where longitudinal expansion occurs, the balloon may expand longitudinally past one or both of the stent ends. Typical stent delivery balloons will expand longitudinally at least 5% beyond the original pre-inflation state. In addition to potentially mis-delivering the medical device as described above, the resulting extended balloon may cause the edges of the stent to push against the vessel wall to a greater extent than they would from radial expansion alone. The protruding stent edges may damage or tear the surrounding vessel resulting in potentially serious trauma for the patient.

It has recently been discovered that Liquid Crystal Polymers (LCP) may be effectively blended with other materials and extruded to form high strength medical balloons. In PCT/US98/18345 there are described medical balloons made from LCP blends.

US Patent No. 5,389,314 to Wang discloses an inflatable medical device which has a plurality of longitudinally oriented conduits which extend throughout the length of the device. The device may be formed by co-extruding two dissimilar plastic materials. The first material form defining a discrete phase which forms fibers and the other material or continuous phase which forms the remaining balloon material. After extrusion the discrete phase is withdrawn from the continuous phase, leaving the continuous phase with a plurality of conduits therethrough.

WO00/50105 is comprised in state of the art according to Art. 54(3, 4) EPC. For this medical device no bulk elongation of a fibril component is specified.

### BRIEF SUMMARY OF THE INVENTION

The present invention is directed generally to medical balloons which expand only to a predetermined extent, and which have minimal longitudinal and/or minimal radial growth during expansion. Specifically, the invention is directed to a stent delivery balloon composed of a micro-composite material which includes a longitudinal fibril structure that is either parallel to the longitudinal axis of the balloon structure, or that is diagonal to the longitudinal axis at the molecular level of the balloon as defined in claim 1. The orientation of the fibril structure can limit longitudinal expansion of the balloon and allow the balloon to expand radially as desired, but minimally, or not at all in the longitudinal direction if the fibrils are parallel to the balloon axis, or when the fibrils are oriented diagonally about the axis, can limit both longitudinal and radial expansion of the balloon when inflated.

The micro-composite material is made up of a combination of a fibril component, a semi-compliant balloon material which acts as a matrix, and optionally a compatibilizer material which may act to create a less distinctive phase boundary between the fibril and matrix components, but which does not solubilize the LCP polymer in the matrix at human body temperature.

The present invention provides for a balloon which utilizes LCP materials or other oriented materials such as PET, in combination with a thermoplastic elastomer matrix and an optional compatibilizer to form a micro-composite material. The present micro-composite material is suitable for construction balloons which exhibit minimal or no longitudinal growth during balloon expansion but which expands as desired in the radial direction, or the present micro-composite material is suitable for construction of balloons that exhibit minimal expansion both in the longitudinal and radial directions.

### BRIEF DESCRIPTION OF THE SEVERAL VIEWS OF THE DRAWINGS

A detailed description of the invention is hereinafter described with specific reference being made to the drawings in which:
FIG. 1 is a schematic representation of side view of a tubular parison used to produce a balloon of the invention from a the micro-fiber composite material;
FIG. 2 is a schematic side view of a medical device delivery balloon constructed from micro-composite material shown at nominal diameter wherein the fibril component is oriented parallel to the longitudinal balloon axis.
FIG. 3 is a view of the medical device delivery balloon shown in Fig. 2 in an inflated state at a pressure higher which causes radial growth of the balloon;
FIG. 4 is a cross-sectional view of a tubular parison for producing balloon of an alternative embodiment of the invention; and
FIG. 5 is a perspective view of the embodiment shown in FIG. 4.
FIG. 6 is perspective view of a dilatation balloon preform in a tubular parison form constructed from micro-composite material wherein the inner and outer fibril components have been oriented diagonally to the longitudinal axis of the tubular preform and in crossing relationship relative to each other by use of a counter-rotating extrusion die.
FIG. 7 is another perspective view of only the outer surface of a dilatation balloon preform constructed from micro-composite material wherein the fibril component is oriented diagonally to the longitudinal axis of the tubular preform by use of a rotating die.
FIG. 8 is a schematic side-view of a blow molded dilatation balloon constructed from micro-composite material depicting the fibril component oriented diagonally to the longitudinal axis of the balloon.

### DETAILED DESCRIPTION OF THE INVENTION

While this invention may be embodied in many different forms, there are shown in the drawings and described in detail herein specific preferred embodiments of the invention. The present disclosure is an exemplification of the principles of the invention and is not intended to limit the invention to the particular embodiments illustrated.

As noted above, the present invention relates to medical catheters which have one or more balloon portions constructed from a specially configured micro-composite material. The particular micro-composite material and configuration provides physical properties which allow a balloon to expand radially to a predetermined extent, but which allow only minimal, or more preferably, no longitudinal growth during expansion. The micro-composite material includes a longitudinal fibril component which exhibits micro-fibers at the molecular level in combination with a matrix of any semi-compliant balloon material. Depending on the specific fibril component, as well as the method of extrusion utilized to extrude the balloon material, the micro-fibers may be randomly scattered through out the balloon material or may be precisely spaced about the balloon and extending through the entire balloon length. The fibril structure is oriented or directed in the longitudinal direction of the balloon providing the balloon with desirable radial expansion characteristics and minimal longitudinal growth when the balloon is inflated.

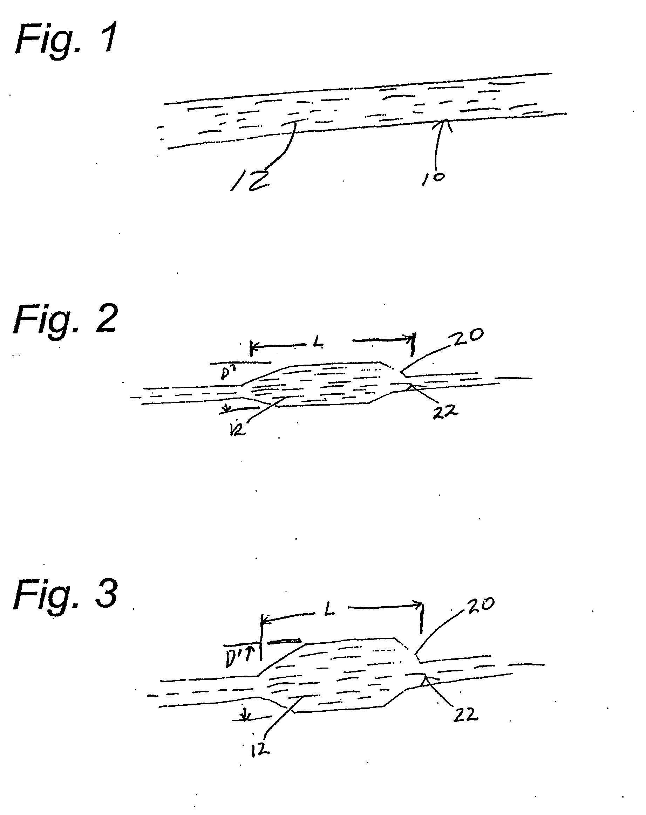

As shown in Fig. 1, the balloons of the invention may be made from tubular parisons 10 of the micro-composite material, having a fibril component which exhibits micro-fibers 12 uniformly oriented in a predetermined direction. In a preferred embodiment shown in Fig. 2, the micro-composite is formed into a balloon 20 from a parison 10 by a conventional balloon blowing process. Balloon 20 has a diameter D and a length L. Micro-fibers 12 are oriented along and about the longitudinal axis 22 of the balloon at the molecular level. The fibril component may be any rigid-rod or semi-rigid-rod thermoplastic material which comprises 0.1 to about 20 percent, and more preferably from about 0.5 to about 15 percent by weight of the micro-composite material. Examples of suitable materials which could be utilized as the fibril component include: liquid crystal polymers such as VECTRA® LKX 1107, 1111, polyetheretherketone (PEEK) material, and PPS. Other materials may also be utilized as the fibril component of the present invention. Such substances include aromatic nylon, rigid polyurethane, polyester, copolyester, polyester blends, polyester/polyurethane blends, PEEK, PPS, fluoropolymer and so on.

To form the micro-composite material, the fibril component is preferably combined with a semi-compliant thermoplastic polymer material in a melt blend which at least partially phase separates upon cooling. Under appropriate conditions the phase separated material will form fibrils or micro-fiber 12 embedded in a matrix of the semi-compliant thermoplastic polymer, oriented substantially parallel to the longitudinal axis of the extruded tubing. The micro-composite material suitably employs an amount of semi-compliant polymer matrix component from about 50 to 99.9 percent by weight, preferably from about 85 to 99.5 percent.

Some examples of suitable materials which may be utilized as the matrix component are polyamide-polyester block copolymers, namely the polyamide/polyether/polyesters PEBAX® 6333, 7033 and 7233; also polyester-polyether block copolymer such as ARINITEL® 540.

As previously described, the present invention achieves the desired balloon expansion characteristics as a result of forming a balloon composed of a micro-composite material. The micro-composite material balloon is formed by coextrusion of a melt blend of LCP or other orientable material, the matrix component, and optionally a compatibilizer. A dual extrusion process utilizing two extruders may also be used to form the desired tube. In the case where LCP is used as the fibril component, the longitudinally oriented fibers are formed by subjecting the blend material to a relatively high extrudate puller speed. The high speed of the puller will subject the blend material to a shearing force which causes a material such as LCP to elongate and form fibers. If the LCP is not subjected to a high shearing force, the LCP will form droplet shaped deposits which provide minimal or no longitudinal stabilization.

If, during extrusion, relative rotation of the mandrel and die is avoided, the fibrils will adopt an orientation substantially parallel to the longitudinal axis. If the die and mandrel are relatively rotated, e/g. by rotation of one or the other or both, the orientation of the fibrils will be helically about the axis.

A balloon which has an LCP fibril component tends to have individual fibers spread randomly throughout the balloon material. The individual LCP fibers will typically be between 0.1 micron to 1 micron in diameter.

If the various components utilized to form the micro-composite material are incompatible to a substantial degree, phase separation may be so efficient that slippage between phases might occur during balloon expansion thereby reducing the longitudinal restriction effect of the fibrils. To prevent such occurrences a compatibilizer may also be desirable for the purpose of enhancing the homogeneity of the melt blend prior to extrusion and cooling. A compatibilizer material may be added to the pre-extruded melt blend material to create a less distinctive phase boundary between the fibril and matrix components. The compatibilizer may be for instance a block copolymer comprising a block which is structurally similar or otherwise is soluble in the matrix polymer and a block which is structurally similar or otherwise soluble with the fibril component. An example of a suitable is the melt compatibilizer disclosed in application US-A-5734352 Such a compatibilizer may be employed in an amount from 0 to about 30 weight percent.

The balloon 20, shown in Fig. 2 at nominal diameter, is shown in Fig. 3 inflated at a higher pressure which provides radial expansion to a new, larger diameter D'. In the most preferred embodiment, the micro-composite material 10 allows balloon 20 to obtain semi-compliant expansion in the radial direction while negating balloon expansion in the longitudinal direction during inflation (balloon length L is substantially unchanged in Fig. 3). Depending on the precise mixture and type of matrix and fibril components used, other embodiments of the present invention may provide for balloons with varying degrees and types of radial expansion while also reducing longitudinal expansion by varying degrees.

If substances less prone to phase separation from the matrix material are desired to be used, an appropriately shaped die may be used in the extrusion process to provide individually extruded fibers evenly around the tube circumference, for instance in the manner of US 5,389,314 except that the fiber material is selected to adhere to the matrix material and a high line speed is used to provide a microscopic fiber diameter. For such an embodiment, the individual non-LCP fibers will typically be between 10 - 12 microns in diameter and may also extend through the entire length of the balloon in chain or cores.

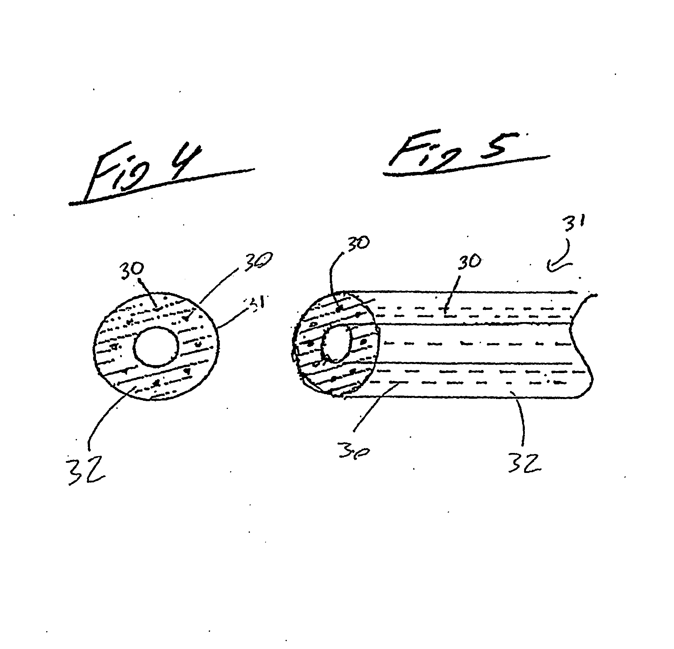

This embodiment is depicted by the tubular parisons in Figs. 4 and 5. As shown, cores 30 are suspended through out the parison 31 in a matrix 32 which may be composed of any material suitable for constructing a semi-compliant balloon as have been described above. The cores 30 are composed of a material which has a more limited ability to stretch than the matrix material, and when the cores are collectively oriented in the same direction, the structures exhibit an increased longitudinal stability when inflated beyond initial or nominal diameter.

In selecting appropriate materials for the fibrils of cores 30 and matrix 32 it is important to select materials which provide adequate adhesion to one another. If adhesion is insufficient between the cores 30 and the surrounding matrix 32 longitudinal growth of the balloon produced from parison 31 will not be restricted as the more expansive matrix material will slip past the individual cores. A further important attribute of the cores 30 is the bulk elongation of the material when oriented as described above. The bulk elongation of the cores 30 should be within the range of 50%-150%. In order to avoid core breakage prior to balloon bursting, it is desirable to the present invention that if the material from which the cores are constructed exhibit a higher tensile strength than the material of which the matrix is constructed.

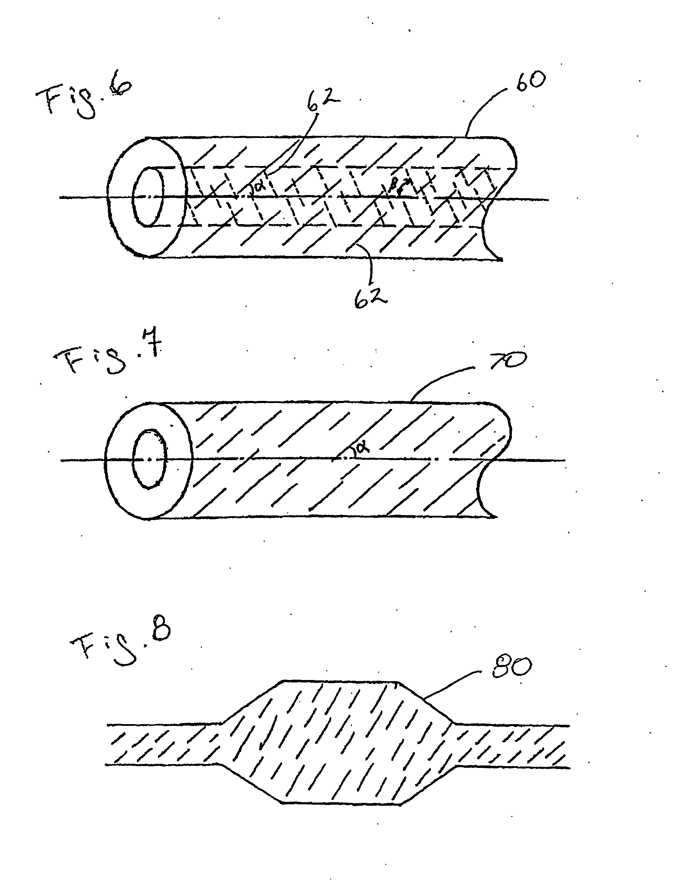

Figures 6-8 pertain to alternative embodiments in which the fibers of the balloon are orientated diagonally relative to the longitudinal axis of the balloon. In Figure 6 there is depicted a parison 60 for a balloon in which, in addition to using a high puller speed during extrusion, a counter rotating die was used. The counter rotating die has a mandrel which rotates in one direction and a concentric outer die which rotates in the opposite direction, the parison is extruded through the space between the two. The resulting parison has fibers 62 orientated diagonally to the parison axis 64 in one direction at the outside surface (angle α) and changing gradually as one passes through the material in a direction transverse to the axis 64 to a second direction (angle β) at the inside surface, the angles determined by outer die/mandrel rotation speeds and puller speed. If one or the other of the outer die or the mandrel are held stationary while the other is rotated, angle α or angle β may be parallel to the axis 64.

In Figure 7 there is depicted a parison 70, having diagonally oriented fibers formed by relative rotation of the die and puller. For instance only the outer die or mandrel may be rotated, so that the fibers become orientated at angle α throughout the entire thickness of the parison.

Figure 8 depicts the outer surface orientation of a balloon 80 made from a parison of either Fig. 6 or Fig. 7. In the balloon body the fibers retain an angular orientation relative to the balloon axis and provide resistance to both longitudinal and radial expansion beyond the nominal or molded dimensions.

Based on the above description it should be understood that several different polymers with a wide range of characteristics may be used to form a longitudinal or longitudinal and radial sfabilized balloon of the present invention. The following is an example of a balloon and its manufacturing parameters which was actually constructed in accordance with the present invention disclosure.

Example 1: a matrix component of Pebax 7033 was mixed with a fibril component of LCP VECTRA LKX 1107 at the ratio of 95 % to 5 % respectively by weight. The mixture was extruded at a rate of (110 feet/minute) 33,5 m/minute line speed into tubing of 0.1 cm (outer diameter) x 0.07 cm (inner diameter [0,039x0,027] inch). A 3,5 mm balloon was formed from the resulting tubing by radial expansion at 110 degrees Celsius with blowing pressure of 24,1 bar (350 psi). The balloon with double wall thickness of 0.004 cm (0.0014 inch) was inflated from 4 atm to 13 atm at 1 atm increment and no measurable balloon length change was observed.

## Claims

1. A dimensionally stable polymer balloon having a longitudinal axis and composed of a micro-composite material, the micro-composite material comprising a polymer matrix component and a liquid crystal polymer fibril component having a diameter of 0.1 micron to 1 micron or having a non liquid crystal polymer fibril component having a diameter of 10 microns to 12 microns distributed in the polymer matrix component, the fibril component oriented parallel or diagonally to the longitudinal axis of the balloon.

2. A catheter having the dimensionally stable polymer balloon of claim 1 mounted thereon.

3. The dimensionally stable polymer balloon of claim 1, wherein said micro-composite material comprises 0.1 wt-% to 20 wt-% of said fibril component.

4. The dimensionally stable polymer balloon of claim 1, wherein said micro-composite material comprises 0.5 wt-% to 15 wt-% of said fibril component.

5. The dimensionally stable balloon of claim 1, wherein said micro-composite material comprises 50 wt-% to 99.9 wt-% of a semi-compliant polymer matrix component.

6. The dimensionally stable balloon of claim 6, wherein said micro-composite material comprises 85 wt-% to 99.5 wt-% of said semi-compliant polymer matrix component.

7. The dimensionally stable balloon of claim 1, wherein said micro-composite material further comprises a compatibilizer component.

8. The dimensionally stable balloon of claim 7 wherein said compatibilizer is a block copolymer.

9. The dimensionally stable balloon of claim 1, wherein said fibril component is composed of rigid-rod thermoplastic material.

10. The dimensionally stable balloon of claim 1, wherein the orientation of said micro-fibers relative to the longitudinal axis of the balloon changes through the balloon material in a direction transverse to said longitudinal axis.

11. A method of forming the dimensionally stable balloon of claim 1, said method comprising the steps of:
(a) melt blending said matrix component and said fibril component forming a mixture wherein said mixture comprises less than about 15 percent by weight but greater than about 0.5 percent by weight of said fibril component, and less than about 99.5 percent by weight but greater than about 85 percent by weight of said matrix component;
(b) forming said mixture into tubing by extrusion in a manner which orients the fibril component along the longitudinal axis of the tubing; and
(c) forming said balloon by radial expansion of a segment of the tubing.

12. The method of claim 11 further comprising a step of adding a compatibilizer to said mixture.

13. A method of forming a balloon as in claim 1, said method comprising the steps of:
a) melt blending said matrix component and said fibril component to form a mixture, wherein said mixture comprises less than about 15 percent by weight but greater than about 0.5 % by weight of said LCP fibril component and less than about 99.5 % by weight but greater than about 85 percent by weight of said matrix component.
b) forming said mixture into a tubular form by extrusion in a manner which orients the fibril component along the longitudinal axis of the tubing;
c) collecting the extruded tube on a puller, wherein the extrudate puller is pulling the tube at a higher speed than the tube is being extruded; and
d) affixing the tubular form onto a catheter.

14. The method of claim 13 further comprising the step of adding a compatibilizer to said mixture.

## Patentansprüche

1. Formstabiler Polymerballon, der eine Längsachse aufweist und aus einem Mikrokomposit-Material besteht, wobei das Mikrokomposit-Material eine Polymermatrix-Komponente und eine LCP-Faserkomponente umfasst, die einen Durchmesser von 0,1 Mikrometer bis 1 Mikrometer aufweist, oder eine Nicht-LCP-Faserkomponente, die einen Durchmesser von 10 Mikrometer bis 12 Mikrometer aufweist und in der Polymermatrix-Komponente verteilt ist, wobei die Faserkomponente parallel oder diagonal zu der Längsachse des Ballons orientiert ist.

2. Katheter, der den formstabilen Polymerballon nach Anspruch 1 befestigt darauf umfasst.

3. Formstabiler Polymerballon nach Anspruch 1, wobei das Mikrokomposit-Material 0,1 Masse-% bis 20 Masse-% der Faserkomponente umfasst.

4. Formstabiler Polymerballon nach Anspruch 1, wobei das Mikrokomposit-Material 0,5 Masse-% bis 15 Masse-% der Faserkomponente umfasst.

5. Formstabiler Polymerballon nach Anspruch 1, wobei das Mikrokomposit-Material 50 Masse-% bis 99,9 Masse-% einer halbelastischen Polymermatrix-Komponente umfasst.

6. Formstabiler Ballon nach Anspruch 6, wobei das Mikrokomposit-Material 85 Masse-% bis 99,5 Masse-% der halbelastischen Polymermatrix-Komponente umfasst.

7. Formstabiler Ballon nach Anspruch 1, wobei das Mikrokomposit-Material weiter eine Haftvermittler-Komponente umfasst.

8. Formstabiler Ballon nach Anspruch 7, wobei der Haftvermittler ein Blockkopolymer ist.

9. Formstabiler Ballon nach Anspruch 1, wobei die Faserkomponente aus einem steifkettigen thermoplastischen Material besteht.

10. Formstabiler Ballon nach Anspruch 1, wobei sich die Orientierung der Mikrofasern im Verhältnis zu der Längsachse des Ballons durch das Ballonmaterial hinweg in einer Richtung verändert, die quer zur Längsachse ist.

11. Verfahren zum Bilden des formstabilen Ballons nach Anspruch 1, wobei das Verfahren die folgenden Schritte umfasst:
(a) Schmelzmischen der Matrixkomponente und der Faserkomponente, wobei eine Mischung gebildet wird, die weniger als etwa 15 Masse-%, aber mehr als etwa 0,5 Masse-% der Faserkomponente umfasst und weniger als 99,5 Masse-%, aber mehr als etwa 85 Masse-% der Matrixkomponente;
(b) Formen der Mischung durch Extrudieren zu einer Röhre, in einer Weise, die die Faserkomponente entlang der Längsachse der Röhre orientiert; und
(c) Bilden des Ballons durch radiale Ausdehnung eines Abschnitts der Röhre.

12. Verfahren nach Anspruch 11, weiter umfassend den Schritt des Hinzufügens eines Haftvermittlers zu der Mischung.

13. Verfahren zum Bilden eines Ballons nach Anspruch 1, wobei das Verfahren die folgenden Schritte umfasst:
(a) Schmelzmischen der Matrixkomponente und der Faserkomponente, um eine Mischung zu bilden, wobei die Mischung weniger als ungefähr 15 Masse-%, aber mehr als 0,5 Masse-% der LCP-Faserkomponente umfasst und weniger als etwa 99,5 Masse-%, aber mehr als etwa 85 Masse-% der Matrixkomponente.
(b) Formen der Mischung durch Extrudieren zu einer röhrenförmigen Form, in einer Weise, die die Faserkomponente entlang der Längsachse der Röhre orientiert;
(c) Auffangen der extrudierten Röhre auf einem Abzieher, wobei der Extrudatabzieher die Röhre mit einer höheren Geschwindigkeit abzieht, als die Röhre extrudiert wird; und
(d) Anbringen der röhrenförmigen Form auf einem Katheter.

14. Verfahren nach Anspruch 13, weiterhin umfassend den Schritt des Hinzufügens eines Haftvermittlers zu der Mischung.

## Revendications

1. Ballonnet polymère stable sur le plan dimensionnel présentant un axe longitudinal et composé d'un matériau microcomposite, le matériau microcomposite comprenant un composant de matrice polymère et un composant en fibrilles de polymère à cristaux liquides présentant un diamètre de 0,1 micron à 1 micron ou comportant un composant en fibrilles de polymère à cristaux liquides ayant un diamètre de 10 microns à 12 microns réparti dans le composant de matrice en polymère, le composant en fibrilles étant orienté de manière parallèle à l'axe longitudinal du ballon ou en diagonale par rapport à celui-ci.

2. Cathéter sur lequel est monté le ballonnet en polymère stable sur le plan dimensionnel selon la revendication 1.

3. Ballonnet en polymère stable sur le plan dimensionnel selon la revendication 1, dans lequel ledit matériau microcomposite comprend de 0,1 % en poids à 20 % en poids dudit composant en fibrilles.

4. Ballonnet en polymère stable sur le plan dimensionnel selon la revendication 1, dans lequel ledit matériau microcomposite comprend de 0,5 % en poids à 15 % en poids dudit composant en fibrilles.

5. Ballonnet stable sur le plan dimensionnel selon la revendication 1, dans lequel ledit matériau microcomposite comprend de 50 % en poids à 99,9 % en poids d'un composant de matrice en polymère semi-flexible.

6. Ballonnet stable sur le plan dimensionnel selon la revendication 6, dans lequel ledit matériau microcomposite comprend de 85 % en poids à 99,5 % en poids d'un composant de matrice en polymère semi-flexible.

7. Ballonnet stable sur le plan dimensionnel selon la revendication 1, dans lequel ledit matériau microcomposite comprend en outre un composant d'agent de compatibilité.

8. Ballonnet stable sur le plan dimensionnel selon la revendication 7, dans lequel ledit agent de compatibilité est un copolymère bloc.

9. Ballonnet stable sur le plan dimensionnel selon la revendication 1, dans lequel ledit composant en fibrilles est composé d'un matériau thermoplastique de tige rigide.

10. Ballonnet stable sur le plan dimensionnel selon la revendication 1, dans lequel l'orientation desdites microfibres par rapport à l'axe longitudinal du ballon change à travers le matériau de ballon dans une direction transversale audit axe longitudinal.

11. Procédé de formation du ballonnet stable sur le plan dimensionnel selon la revendication 1, ledit procédé comprenant les étapes consistant à :
(a) mélanger par fusion ledit composant de matrice et ledit composant en fibrilles pour former un mélange dans lequel ledit mélange comprend moins d'environ 15 pourcent en poids mais plus d'environ 0,5 pourcent en poids dudit composant en fibrilles et moins d'environ 99,5 pourcent en poids mais plus d'environ 85 pourcent en poids dudit composant de matrice ;
(b) former ledit mélange en un tube par extrusion d'une manière qui oriente le composant en fibrilles le long de l'axe longitudinal du tube ; et
(c) former ledit ballon par expansion radiale d'un segment du tube.

12. Procédé selon la revendication 11, comprenant en outre une étape d'ajout d'un agent de compatibilité dans ledit mélange.

13. Procédé de formage d'un ballon selon la revendication 1, ledit procédé comprenant les étapes consistant à :
(a) mélanger par fusion ledit composant de matrice et ledit composant en fibrilles pour former un mélange, dans lequel ledit mélange comprend moins d'environ 15 pourcent en poids mais plus d'environ 0,5 pourcent en poids dudit composant en fibrilles de LCP et moins d'environ 99,5 % en poids mais plus d'environ 85 pourcent en poids dudit composant de matrice ;
(b) former ledit mélange en une forme tubulaire par extrusion, de manière à orienter le composant en fibrilles le long de l'axe longitudinal du tube ;
(c) collecter le tube extrudé sur une tireuse, dans laquelle la tireuse d'extrudat tire le tube à une vitesse supérieure à la vitesse d'extrusion du tube ; et
(d) fixer la forme tubulaire sur un cathéter.

14. Procédé selon la revendication 13, comprenant en outre l'étape consistant à ajouter un agent de compatibilité audit mélange.
